# EUROPEAN PATENT SPECIFICATION

(11) **EP 1 795 532 B1**
(45) Date of publication and mention of the grant of the patent: **11.03.2009**
(21) Application number: 06124559.3
(22) Date of filing: 22.11.2006
(51) Int. Cl.: C07D 471/22, G03G 5/00

(54) **Multicyclic benzimidazole derivatives useful as photoconductive members**
Multizyklische Benzimidazol-Derivate verwendbar als fotoleitfähige Elemente
Dérivés de benzimidazole multicycliques et leur utilisation comme des éléments photoconducteurs

(30) Priority: 12.12.2005 US 301217
(43) Date of publication of application: 13.06.2007
(73) Proprietor: Xerox Corporation, Rochester, NY 14644 (US)
(72) Inventor: Duff, James M., RR3, Orillia, Ontario L3V 6H3 (CA); Bender, Timothy P., Toronto Ontario M8Y 3Y9 (CA); Vong, Cuong, Hamilton Ontario L9C 3H5 (CA); Graham, John F., Oakville Ontario L6M 4S1 (CA)
(74) Representative: Grünecker, Kinkeldey, Stockmair & Schwanhäusser Anwaltssozietät

(56) References cited:
- US-A- 4 808 506
- US-A- 5 139 909
- DATABASE WPI Week 200515 Derwent Publications Ltd., London, GB; AN 2005-136342 XP002426106 -& JP 2005 033185 A (MITSUBISHI CHEM CORP) 3 February 2005 (2005-02-03)
- LOUTFY R O ET AL: "ORGANIC PHOTOCONDUCTIVE (OPC) DEVICES INCORPORATING BISARYLIMIDAZOLE PERINONE PIGMENTS" DYES AND PIGMENTS, ELSEVIER APPLIED SCIENCE PUBLISHERS. BARKING, GB, vol. 15, no. 2, January 1991 (1991-01), pages 139-156, XP000179032 ISSN: 0143-7208
- DATABASE CA [Online] CHEMICAL ABSTRACTS SERVICE, COLUMBUS, OHIO, US; WATANABE, KOICHI ET AL: "Naphthoylenedibenzimidazole dyes" XP002425909 retrieved from STN Database accession no. 1977:191334 & JP 52 024225 A (YAMAMOTO SYNTHETIC CHEMICAL CO., LTD., JAPAN) 23 February 1977 (1977-02-23)
- DATABASE CA [Online] CHEMICAL ABSTRACTS SERVICE, COLUMBUS, OHIO, US; PLAKIDIN, V.L. ET AL.: "Naphthoylenebenzimidazole dyes" XP002425910 retrieved from STN Database accession no. 1967:19835 & COLLECTION OF CZECHOSLOVAK CHEMICAL COMMUNICATIONS , 30(11), 3718-29 CODEN: CCCCAK; ISSN: 0010-0765, 1965,
- DATABASE CA [Online] CHEMICAL ABSTRACTS SERVICE, COLUMBUS, OHIO, US; ARIENT, J. ET AL: "Imidazole dyes. XV. Synthesis of aroyleneimidazole dyes and influence of substitution on their dyeing properties" XP002425911 retrieved from STN Database accession no. 1966:20015 & IZOBRET., PROM. OBRAZTSY, TOVARNYE ZNAKI 1966, 43(12), 66CODEN: URXXAF, 1965,

## Description

The present invention relates to a photoconductive member component comprising a supporting substrate and thereover a photogenerating layer comprising a specific perinone derivative. The invention further relates to an image forming apparatus comprising said photoconductive member component.

Photoconductive imaging members containing the aforementioned components possess a number of advantages as indicated herein, inclusive of being sensitive to blue wavelengths. The photogenerating layer, which can be exposed to light of the appropriate blue wavelengths simultaneously, or sequentially, exhibits, for example, excellent cyclic stability, independent layer discharge, acceptable dark decay characteristics, permits tuning of the electrical properties of the imaging member, and enables substantially no adverse changes in performance over extended time periods.

The layered photoconductive imaging members illustrated herein can be selected for a number of different known imaging and printing processes including, for example, multicopy/fax devices, electrophotographic imaging processes, especially xerographic imaging and printing processes wherein negatively charged or positively charged images are rendered visible with toner compositions of an appropriate charge polarity. embodiments can be selected for color xerographic imaging applications where several color printings can be achieved in a single pass.

Photoconductive or photoresponsive imaging members are disclosed in the following U. S. Patents: U. S. Pat. No. 4,265,990,4,419,427, 4,429,029, 4,501,906, 4,555,463, 4,587,189, 4,709,029, 4,714,666, 4,937,164, 4,968,571, 5,019,473, 5,225,307, 5,336,577, 5,473,064, 5,645,965, 5,756,245, 6,051,351, 6,194,110, and 6,656,651.

Photoconductive imaging members comprising a cis-type or trans-type perinone derivative are known from U. S. Patents Nos. 4,808,506 and 5,139,909, and R. O. Loutfy et al., "Organic Photoconductive (OPC) Devices Incorporating Bisarylimidazole Perinone Pigments", Dyes and Pigments, Elsevier Applied Science Publishers, Barking, GB, vol. 15, no. 2, January 1991, pages 139-156.

The present invention provides a photoconductive member component comprising a supporting substrate and thereover a photogenerating layer comprising a perinone derivative selected from the following compounds:

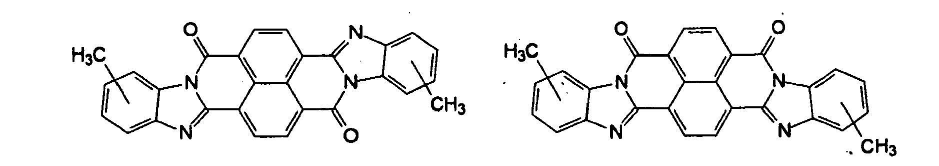

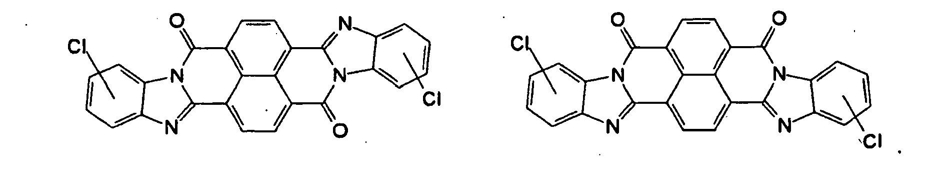

Moreover, the present invention provides an image forming apparatus for forming images on a recording medium comprising:
a) a photoreceptor member having a charge retentive surface to receive an electrostatic latent image thereon, wherein said photoreceptor member comprises the above photoconductive member component;
b) a development component to apply a developer material to said charge-retentive surface to develop said electrostatic latent image to form a developed image on said charge-retentive surface;
c) a transfer component for transferring said developed image from said charge-retentive surface to another member or a copy substrate; and
d) a fusing member to fuse said developed image to said copy substrate.

Imaging members are provided with many of the advantages illustrated herein, including, for example, photoresponsive imaging members with excellent photosensitivity to blue light radiations, layered photoresponsive imaging members with a sensitivity to blue light, and which members possess in embodiments tunable and preselected electricals, acceptable dark decay characteristics, and high photosensitivity. Morever, provided are improved layered photoresponsive imaging members with photosensitivity to blue light, for example, in the wavelength region of from 350 to 450 nanometers or more specifically 370 to 425 nanometers. The photoresponsive or photoconductive imaging members disclosed can be selected for imaging processes including for example xerography.

The bisbenzamidazoleperinones used in the invention are those of the following formulas: 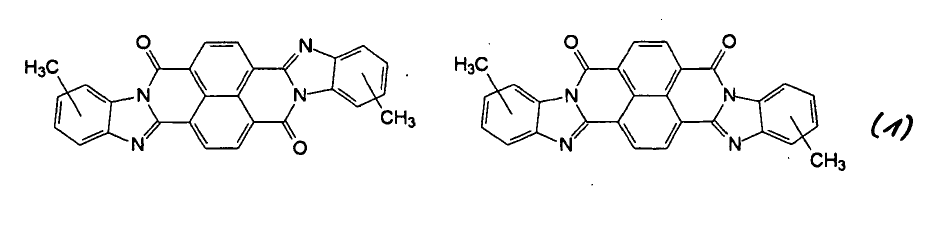 representing a mixture of products obtained by the condensation of 1,4,5,8-naphthalene tetracarboxylic anhydride with 3,4-diaminotoluene; and 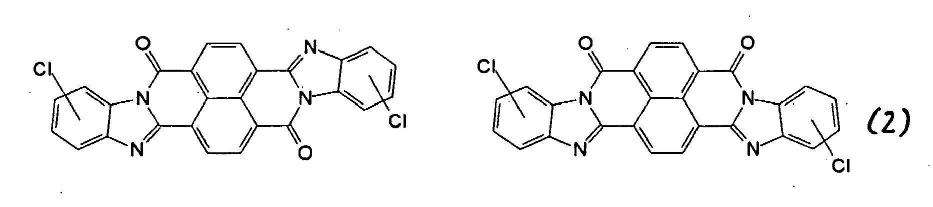 representing a mixture of products obtained by the condensation of 1,4,5,8-naphthalene tetracarboxylic anhydride with 3,4-diaminochlorobenzene.

The bisbenzamidazoleperinones can be prepared by a number of methods such as the reaction of a 1,4, 5, 8-naphthalene tetracarboxylic dianhydride with a 1,2-arylene diamine to form a crude product, which may or may not be isolated and/or purified, followed by a process such as crystallization and/or train sublimation to provide the photogenerator component.

For example, the compounds may be prepared by a one-step, one-pot reaction of a 1,4,5,8-naphthalene tetracarboxylic anhydride with an equal molar amount (to the anhydride group) or slight molar excess (to the anhydride group) of a 1,2-diaminoarylene compound at temperatures between 150 °C to 200 °C in a suitably high boiling polar solvent such as N-methylpyrrolidone, N,N-dimethylacetamide, hexamethylphosphoramide, m-cresol and the like, and usually in the presence of a catalyst selected in an amount of for example between 1 mol % to 10 mol %, such as salts of zinc, aluminum, iron, gallium, tin and the like. After a certain period of time at reaction temperature, the reaction mixture is cooled and usually diluted with an alcohol such as isopropanol. The crude product, which is usually insoluble in alcohol, can be isolated by common filtration techniques. A process to purify the compound prior to its utilization as a photogenerator can be selected, such as, for example, fractional or train sublimation and/or crystallization from a suitable solvent and/or stirring in either a hot or cold solvent suitable for dissolution of unwanted impurities.

In embodiments, there is provided a member wherein the photogenerating layer is of a thickness of from 0.1 to 60 or 1 to 30 microns; a member wherein the photogenerator component amount is from 0.05 weight percent to 90 weight percent or from 20 weight percent to 90 weight percent of binder, and wherein the total of the components is about 100 percent; and wherein the photogenerator component is dispersed in from 10 to 75 weight percent of a polymer binder; a member that absorbs light of a wavelength of from 350 to 450 nanometers or 370 to 425 nanometers; an imaging member wherein the supporting substrate is comprised of a conductive substrate comprised of a metal; an imaging member wherein the conductive substrate is aluminum, aluminized polyethylene terephthalate or titanized polyethylene terephthalate or a metalized plastic film wherein the metal layer may be comprised of a single metal or a mixture of metals and wherein the plastic film may be any film of suitable mechanical properties so as to act as a supporting substrate; an imaging member wherein the photogenerator binder is selected from the group consisting of polyesters, polyvinyl butyrals, polycarbonates, polystyrene-b-polyvinyl pyridine, and polyvinyl formyls; an imaging member wherein the charge transport layer is a hole transporting layer comprised of arylamine molecules and wherein such a layer is transparent to radiation at between 350 to 450 nanometers or 370 to 425 nanometers; a method of imaging which comprises generating an electrostatic latent image on the imaging member of the present disclosure, developing the latent image, and transferring the developed electrostatic image to a suitable substrate; a method of imaging wherein the imaging member is exposed to light of a wavelength of from 350 to 450 nanometers or 370 to 425 nanometers; an imaging apparatus containing a charging component, a development component, a transfer component, and a fixing component and wherein the apparatus contains a photoconductive imaging member comprised of supporting substrate, and thereover a layer comprised of the bisbenzamidazoleperinone photogenerating pigment and a hole transport layer; an imaging apparatus containing a charging component, a development component, a transfer component, and a fixing component, and wherein the apparatus contains a photoconductive imaging member comprised of supporting substrate, and thereover a component as described herein, wherein the component is a photoconductor; an imaging member further containing an adhesive layer and a hole blocking layer; an imaging member wherein the blocking layer is contained as a coating on a substrate and wherein the adhesive layer is coated on the blocking layer; an imaging member further containing an adhesive layer and a hole blocking layer; a method of imaging which comprises generating an electrostatic latent image in the imaging member of the present disclosure; developing the latent image, and transferring the developed electrostatic image to a suitable substrate; and a color method of imaging which comprises generating an electrostatic latent image on the imaging member, developing the latent image, transferring and fixing the developed electrostatic image to a suitable substrate; and photoconductive imaging members with the bisbenzamidazoleperinone photogenerating component.

In embodiments, the photogenerating layer can be selected at a thickness of from 0.1 to 60 or 1 to 30 microns, the charge transport layer can be selected at a thickness of from 5 to 200 microns, 10 to 100 microns, or 15 to 30 microns and each of the layers can be selected to contain from 10 weight percent to 75 weight percent of a polymer binder, the photogenerating layer can be selected in an amount of from 10 to 70 weight percent, and the binder can be selected in an amount of 30 to 90 weight percent.

The photogenerating components and the charge transport components are in embodiments dispersed in a suitable binder, for example a polymer binder, such as for example, polycarbonates, polyesters, polyvinylbutyral, polysiloxanes and polyurethanes. The photogenerating pigments can be present in various amounts, such as, for example, from 0.05 to 90 weight percent, from 10 to 90 weight percent, or from 15 to 50 weight percent and the polymer binder can be present in an amount of from 10 to 90 weight percent, 25 weight percent to 75 weight percent, or 25 to 50 weight percent. The thickness of this layer can be, for example, from 0.1 microns to 60 microns or from 1 micron to 30 microns.

There can also be selected for members of the present disclosure a suitable adhesive layer, which can be for example situated between the substrate and the single layer, examples of adhesives being polyesters, such as VITEL® PE 100 and PE 200 available from Goodyear Chemicals or MOR-ESTER 49,0000® available from Norton International. This adhesive layer can be coated on to the supporting substrate from a suitable solvent, such as tetrahydrofuran and/or dichloromethane solution, to enable a thickness thereof ranging, for example, from 0.001 to 5 microns, and more specifically, from 0.1 to 3 microns.

The photoconductive imaging members can be economically prepared by a number of methods, such as the coating of the components from a dispersion, and more specifically, as illustrated herein. Thus, the photoresponsive imaging member disclosed herein can in embodiments be prepared by a number of known methods, the process parameters being dependent, for example, on the member desired. The photogenerating and charge transport components for the imaging members can be coated as solutions or dispersions onto a selected substrate by the use of a spray coater, dip coater, extrusion coater, roller coater, wire-bar coater, slot coater, doctor blade coater, gravure coater, and the like, and dried for example at a temperature of from 40°C to 200 °C for a suitable period of time, such as from 10 minutes to 10 hours under stationary conditions or in an air flow. The coating can be accomplished to provide a final coating thickness of for example from 0.01 to 30 microns after drying. The fabrication conditions for a given photoconductive layer can be tailored to achieve optimum performance and cost in the final members. The coating in embodiments can also be accomplished with spray, dip or wire-bar methods such that the final dry thickness of the photogenerating layer is, for example, from 0.1 to 50 microns, or 1 to 10 microns after being dried at, for example, 40 °C to 150 °C for example for 5 to 90 minutes.

Examples of substrate layers selected for the present imaging members can be opaque or substantially transparent, and can comprise any suitable material having the requisite mechanical properties. Thus, the substrate can comprise a layer of insulating material including inorganic or organic polymeric materials, such as MYLAR®, a commercially available polymer, MYLAR® containing titanium, a layer of an organic or inorganic material having a semiconductive surface layer, such as indium tin oxide, or aluminum arranged thereon, or a conductive material inclusive of, but not limited to, aluminum, chromium, nickel, titanium, zirconium, brass or the like. The substrate may be flexible, seamless, or rigid, and may have a number of many different configurations, such as, for example, a plate, a cylindrical drum, a scroll, an endless flexible belt, and the like. In one embodiment, the substrate is in the form of a seamless flexible belt. In some situations, it may be desirable to coat on the back of the substrate, such as when the substrate is a flexible organic polymeric material, an anticurl layer, such as, for example, polycarbonate materials commercially available as MAKROLON®.

The thickness of the substrate layer depends on many factors, including economical considerations, thus this layer can be of substantial thickness, for example, over 3,000 microns, or of a minimum thickness. In one embodiment, the thickness of this layer is from 75 microns to 300 microns.

Generally, the thickness of the layer in contact with the supporting substrate depends on a number of factors, including the thickness of the substrate, and the amount of components contained in the single layer, and the like. Accordingly, the layer can be of a thickness of, for example, from 0.1 micron to 50 microns, and more specifically, from 1 micron to 10 microns. The maximum thickness of the layer in embodiments is dependent primarily upon factors, such as photosensitivity, electrical properties and mechanical considerations. The binder resin can be selected in various suitable amounts, for example, from 5 to 70, and more specifically, from 10 to 50 weight percent, and can comprise a number of known polymers such as poly(vinyl butyral), poly(vinyl carbazole), polyesters, polycarbonates, poly(vinyl chloride), polyacrylates and methacrylates, copolymers of vinyl chloride and vinyl acetate, phenoxy resins, polyurethanes, poly(vinyl alcohol), polyarylonitrile, polystyrene, and the like. In embodiments, single layer coating solvents selected can include, for example, ketones, alcohols, aromatic hydrocarbons, halogenated aliphatic hydrocarbons, ethers, amines, amides, esters, and the like. Specific examples include, but are not limited to, cyclohexanone, acetone, methyl ethyl ketone, methanol, ethanol, butanol, amyl alcohol, toluene, xylene, chlorobenzene, carbon tetrachloride, chloroform, methylene chloride, trichloromethylene, tetrahydrofuran, dioxane, diethyl ether, dimethyl formamide, dimethyl acetamide, butyl acetate, ethyl acetate, methoxyethyl acetate, and the like.

As optional adhesives usually in contact with the supporting substrate, there can be selected various known substances inclusive of polyesters as indicated herein, polyamides, poly(vinyl butyral), poly(vinyl alcohol), polyurethane and polyacrylonitrile. This layer is of a suitable thickness, for example a thickness of from 0.001 micron to 25 microns. Optionally, this layer may contain effective suitable amounts, for example from 1 to 10 weight percent, of conductive and nonconductive particles, such as zinc oxide, titanium dioxide, silicon nitride, carbon black, an the like, to provide, for example, in embodiments, further desirable electrical and optical properties.

Aryl amines selected for the hole transporting layer in contact with the photogenerating layer include molecules of the following formula 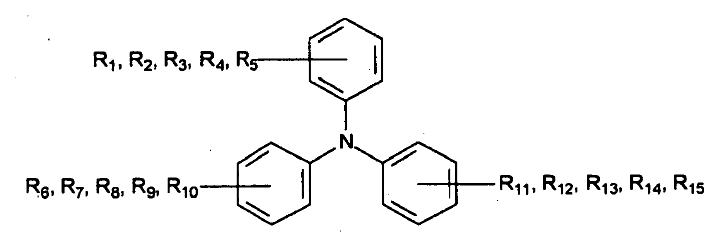 where R₁ through R₁₅ are independently chosen from the group alkyl, substituted alkyl, alkoxy, alkoxylalkyl, phenyl, naphthyl and higher aromatic compounds such as anthracene, other fused aromatic ring systems such as carbazole, stilbene and the like, halogen and hydrogen. Each of R₁ through R₁₅ can be selected to have a total atom count of between 1 and 50, between 1 and 10 or between 1 and 5. R₁ through R₁₅ can be selected in such a way that at least one of R₁ through R₁₅ is alkoxy, for example, methoxy, or alkyl, for example, methyl. A selected embodiment comprises bis(3,4-dimethylphenyl)-4-methoxphenyl amine) or tri-toylamine. Another selected embodiment comprises dimers of the above but not of the benzidine type, for example 1,1-bis (di-4-tolylaminophenyl) cyclohexane. In yet another embodiment, example mixtures of arylamine compounds can be used for example mixtures of tri-tolylamine and 1,1-bis (di-4-tolylaminophenyl) cyclohexane.

Other known charge transport molecules can be selected, reference for example, U.S. Patent Nos. 4,921,773 and 4,464,450.

Polymer binder examples for the hole transport molecules include components as illustrated, for example, in U.S. Pat. No. 3,121,006. Specific examples of polymer binder materials include polycarbonates, acrylate polymers, vinyl polymers, cellulose polymers, polyesters, polysiloxanes, polyamides, polyurethanes, and epoxies as well as block, random, or alternating copolymers thereof. Specifically, electrically inactive binders can be selected comprised of polycarbonate resins with a molecular weight of from 20,000 to 100,000 or more specifically a with a molecular weight of from 50,000 to 100,000.

Further included are methods of imaging and printing with the photoresponsive or photoconductive members illustrated herein. These methods generally involve the formation of an electrostatic latent image on the imaging member, followed by developing the image with a toner composition comprised, for example, of thermoplastic resin, colorant, such as pigment, charge additive, and surface additives, reference for example U.S. Pat. Nos. 4,560,635; 4,298,697; and 4,338,380, subsequently transferring the image to a suitable substrate, and permanently affixing, for example, by heat, the image thereto. In those environments wherein the member is to be used in a printing mode, the imaging method is similar with the exception that the exposure step can be accomplished with a laser device or image bar.

### EXAMPLES

The following Examples are being submitted to further define various species of the present disclosure. Parts and percentages are by weight unless otherwise indicated.

### Example 1

1,4,5,8-naphthalene tetracarboxylic dianhdyride (1 equiv), 3,4-diaminotoluene (2.5 equiv) and zinc(II)acetate (5 mol %) were heated to reflux in N-methyl-1,2-Pyrrolidone (NMP) (10 wt % solids) for 5 hours, cooled to room temperature and filtered. The filter cake was washed with N,N-dimethylformamide (DMF) (3 washes of 20 milliliters each wash) and methanol (3 washes of 20 milliliters each wash) and dried at about 80 °C under vacuum of about 10 millimeters mercury overnight to yield 2.5 grams of bisbenzamidazoleperinone having the structure (1). The 2.5 grams of bisbenzamidazoleperinone was purified by train sublimation as known to those skilled in the art (for example as described in H. J. Wagner, R. O. Loutfy and C.-K. Hsaio, J. Mater. Sc. 17, 2781, 1982) to yield 2 grams of bisbenzamidazoleperinone whose purity and absolute identity was confirmed using primarily 1H nuclear magnetic resonance spectroscopy (using CDCl₃/TFA-d 3/1 v/v (a mixture of deuterated chloroform and deuterated trifluoroacetic acid mixed in a ratio of 3:1 by volume) as the solvent and tetramethylsilane (TMS) as an internal standard) and elemental analysis.

### Example 2

### Preparation of evaporated pigment generator layer

Thin film of 5000 Å was prepared by vacuum evaporation in a Balzer BAE080™ coater. Compounds as described in Example 1 were loaded into a tantalum boat, and then capped after filling. The system pressure remained stable at < 10⁻⁵ mm Hg during the evaporation. The boat was gradually heated until it reached the temperature where the pigment began to sublime. The pigment vapor deposited onto a titanized MYLAR® substrate of 75 microns in thickness which substrate contained thereon a silane layer, 0.1 micron in thickness, situated above the source at a control rate of 2-4 Å/s, as monitored by a Quartz crystal monitor.

### Example 3

### Preparation of binder generator layer

0.2 gram of compounds as described in Example 1 were mixed with 0.05 gram of poly-N-vinylcarbazole (PVK) and 10.5 grams dichloromethane in a 30 milliliter glass bottle containing 70 grams 1/8" stainless steel shots, then placed on a roll mill for 3 days with gentle to moderate rolling. Using a film applicator with a gap of 1.5 mil, the pigment dispersion was coated on a titanized MYLAR® substrate of 75 microns in thickness which substrate contained thereon a silane layer, 0.1 micron in thickness. Thereafter, the photogenerator layer formed was dried in a forced air oven at 135 °C for 20 minutes.

### Example 4

### Preparation of hole transport layer

A transport layer solution was prepared by mixing 2.025 grams of polycarbonate (PC(Z)400), 0.675 grams of tritoylamine, 0.675 grams of 1,1-bis-(N,N-ditoyl-4-aminophenyl) cyclohexane and 15.38 grams of methylene chloride. The resulting solution was coated onto the above photogenerating layer using a film applicator of 10 mil gap. The resulting photoconductive member was then dried at 135 °C in a forced air oven for 20 minutes. The final dried thickness of the transport layer was 25 microns.

### Example 5

### Electrical measurements of device

The xerographic electrical properties of the above-prepared photoconductive imaging members and other similar members can be determined by known means, including electrostatically charging the surfaces thereof with a corona discharge source until the surface potentials, as measured by a capacitively coupled probe attached to an electrometer, attained an initial value Vo of about -800 volts. After resting for 0.5 second in the dark, the charged members attained a surface potential of V_{ddp}, dark development potential. Each member was then exposed to light from a filtered Xenon lamp thereby inducing a photodischarge which resulted in a reduction of surface potential to a V_{bg} value, background potential. The percent of photodischarge was calculated as 100x(V_{ddp} - V_{bg})N_{ddp}. The desired wavelength and energy of the exposed light was determined by the type of filters placed in front of the lamp. The monochromatic light photosensitivity was determined using a narrow band-pass filter. The photosensitivity of the imaging member was usually provided in terms of the amount of exposure in ergs/cm², designated as E_{1/2}, required to achieve 50 percent photodischarge from V_{ddp} to half of its initial value. The higher the photosensitivity, the smaller is the E_{1/2} value. The device was finally exposed to an erase lamp of appropriate light intensity and any residual potential (V_{residual}) was measured. The imaging members were tested with an exposure monochromatic light at a wavelength of 400 nanometers and an erase broad-band light with the wavelength of 400 to 800 nanometers.

### Comparative Example 1

Procedure identical to that described in Example 1 except 3,4-diaminotoluene was replaced by an equivalent amount (based on moles) of 3,4-dimethyl-1,2-phenylene diamine.

### Comparative Example 2

Procedure identical to that described in Example 1 except 3,4-diaminotoluene was replaced by an equivalent amount (based on moles) of 2,3-diaminonaphthalene.

### Comparative Example 3

Procedure identical to that described in Example 1 except 3,4-diaminotoluene was replaced by an equivalent amount (based on moles) of 4-chloro-1,2-phenylene diamine.

### Comparative Example 4

Procedure identical to that described in Example 1 except 3,4-diaminotoluene was replaced by an equivalent amount (based on moles) of 1,2-phenylene diamine.

**Table 1**

| Pigment | Example #/ Sample ID | DD | S | E1/2 | E7/8 | Vr |
|---|---|---|---|---|---|---|
| | | (500ms)(-V) | (Verg/cm²) | (ergs/cm²) | (ergs/cm²) | (-V) |
| Example 1 bis(methylbenzimidazo)perinone | 1 | 2 | 85 | 5.54 | 12.29 | 17 |
| Example 2 bis(methylbenzimidazo)perinone | 2 | 2 | 76 | 6.16 | - | 14 |
| Comparative Example 1 bis(dimethylbenzimidazo)perinone | 3 | 15 | 62 | 7.82 | - | 17 |
| Comparative Example 2 bis(2,3-naphthimidazo)perinone | 4 | 5 | 49 | 9.66 | - | 14 |
| Comparative Example 3 bis(4-chlorobenzimidazo)perinone | 5 | 2 | 47 | 9.82 | - | 24 |
| Comparative Example 4 bis(benzimidazo)perinone | 6 | 2 | 31 | 11.76 | - | 7 |
| where DD = dark decay; S = sensitivity; E_{1/2} = exposure to decrease charge to ½ initial value; E_{7/8} = exposure to decrease charge to 7/8 initial value; and Vr = residual potential | | | | | | |

A photoconductive imaging member fabricated by the process of Example 4 using the pigment of Example 1 had a dark decay of 2 volts/second, a sensitivity of 85 Verg/cm², an E_{1/2} of 5.54 ergs/cm² and the V_{residual} was 17 volts for negative charging. The member was sensitive to blue light of a wavelength of 400 nanometers, and which wavelength was generated from a 400 nanometer single-band pass filter placed in front of a xenon lamp.

A photoconductive imaging member fabricated by the process of Example 4 using the pigment of Example 2 had a dark decay of 2 volts/second, a sensitivity of 76 Verg/cm² and the V_{residual} was 14 volts for negative charging. The member was sensitive to blue light of a wavelength of 400 nanometers, and which wavelength was generated from a 400 nanometer single-band pass filter placed in front of a xenon lamp.

## Claims

1. A photoconductive member component comprising a supporting substrate and thereover a photogenerating layer comprising a perinone derivative selected from the following compounds:

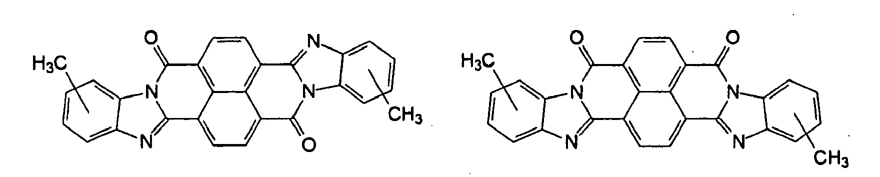

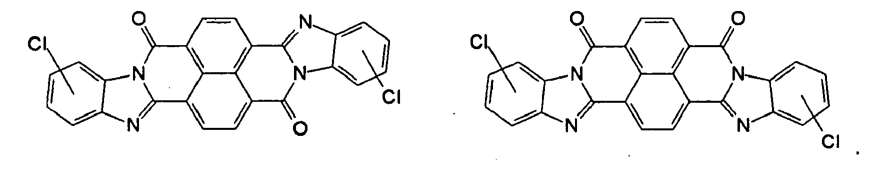

2. An image forming apparatus for forming images on a recording medium comprising:
a) a photoreceptor member having a charge retentive surface to receive an electrostatic latent image thereon, wherein said photoreceptor member comprises the photoconductive member component of claim 1;
b) a development component to apply a developer material to said charge-retentive surface to develop said electrostatic latent image to form a developed image on said charge-retentive surface;
c) a transfer component for transferring said developed image from said charge-retentive surface to another member or a copy substrate; and
d) a fusing member to fuse said developed image to said copy substrate.

## Patentansprüche

1. Komponente eines fotoleitfähigen Elementes, umfassend ein Trägersubstrat und darüber eine fotoerzeugende Schicht, umfassend ein Perinonderivat, das aus den folgenden Verbindungen ausgewählt ist:

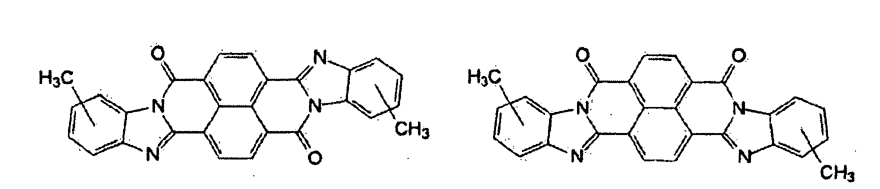

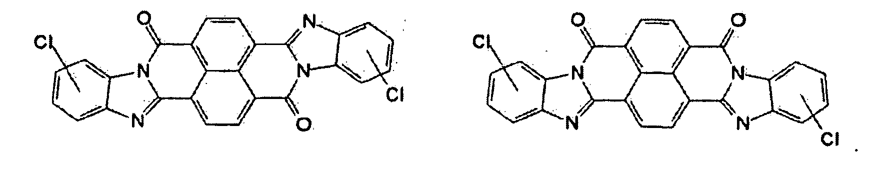

2. Bilderzeugungsapparat zum Erzeugen von Bildern auf einem Aufzeichnungsmedium, umfassend:
a) ein Fotorezeptorelement mit einer Ladung zurückhaltenden Oberfläche, um ein elektrostatisches Latentbild darauf zu empfangen, wobei das Fotorezeptorelement die Komponente eines fotoleitfähigen Elementes nach Anspruch 1 umfasst;
b) eine Entwicklungskomponente, um ein Entwicklermaterial auf die Ladung zurückhaltende Oberfläche aufzubringen, um das elektrostatische Latentbild zu entwickeln, um ein entwickeltes Bild auf der Ladung zurückhaltenden Oberfläche zu bilden;
c) eine Übertragungskomponente zum Übertragen des entwickelten Bildes von der Ladung zurückhaltenden Oberfläche auf ein anderes Element oder ein Kopiersubstrat; und
d) ein Schmelzfixierelement, um das entwickelte Bild auf das Kopiersubstrat aufzuschmelzen.

## Revendications

1. Composant d'élément photoconducteur comprenant un substrat de support et par-dessus celui-ci une couche photogénératrice comprenant un dérivé de périnone choisi parmi les composés suivantes :

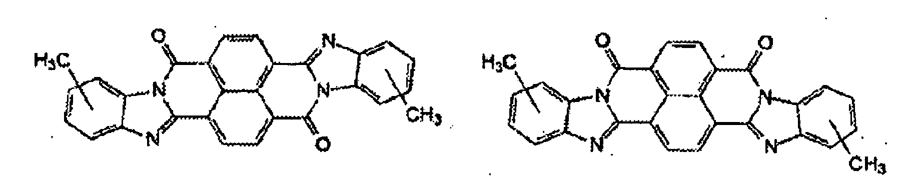

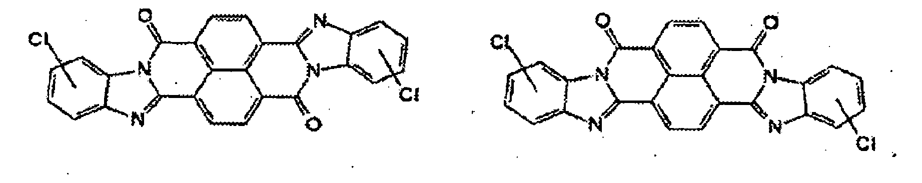

2. Appareil de formation d'images pour former des images sur un support d'enregistrement comprenant :
a) un élément photorécepteur ayant une surface de rétention de charge pour recevoir une image latente électrostatique sur celle-ci, dans lequel ledit élément photorécepteur comprend le composant d'élément photoconducteur selon la revendication 1 ;
b) un composant de révélation pour appliquer une matière de révélateur à ladite surface de rétention de charge pour révéler ladite image latente électrostatique pour former une image révélée sur ladite surface de rétention de charge ;
c) un composant de transfert pour transférer ladite image révélée de ladite surface de rétention de charge sur un autre élément ou un substrat de copie ; et
d) un élément de fusion pour fusionner ladite image révélée audit substrat de copie.
